# EUROPEAN PATENT APPLICATION

(11) **EP 4 542 212 A1**
(43) Date of publication of application: **23.04.2025**
(21) Application number: 23203800.0
(22) Date of filing: 16.10.2023
(51) Int. Cl.: G01N 27/327, C12Q 1/00

(54) **ELECTROCHEMICAL BIOSENSOR OF METABOLITES FOR IN VITRO INVESTIGATIONS IN BIOLOGICAL SAMPLES**

(71) Applicant: Vilnius University, 01513 Vilnius (LT)
(72) Inventor: Razumiene, Julija, LT-03102 Vilnius (LT); Gureviciene, Vidute, LT-06118 Vilnius (LT); Sakinyte-Urbikiene, Ieva, LT-14300 Pikutiskiu k., Vilniaus raj. (LT); Dagys, Marius, LT-27122 Vilnius (LT); Ratautas, Dalius, LT-08409 Vilnius (LT)
(74) Representative: Klimaitiene, Otilija

(57) **Abstract**

The invention discloses an enzyme- and carbonaceous material (e.g. thermallyreduced graphene oxide (RGO))-based amperometric biosensor for direct evaluation of selected metabolites in biological media such as brain extract or serum. The biosensor comprises a working electrode (2), and a permeable membrane (1) covered with immobilized metabolite-selective enzyme with the carbonaceous material, being in contact with the electrode (2) surface, for bioelectrocatalytic oxidation of the metabolite, thus measuring its presence. The determination of a specific metabolite - L-glutamate - is based on registration at -0.1 V (*vs.* Ag/AgCl) of anodic current generated by the electrooxidation of ammonia released during the reaction catalyzed by L-glutamate oxidase, while L-glutamate is converting to alfa-ketoglutarate. The biosensor comprises a working graphite electrode (2) and adjustable semipermeable membrane (1) comprising L-glutamate oxidase (EC 1.4.3.11) immobilized into albumin and thermally reducated graphene oxide (RGO).

## Description

### TECHNICAL FIELD

The invention relates to a biosensor for measurements of metabolites in biological samples, the biosensor having a special construction with an enzymatic membrane. An embodiment of the invention is disclosed: an oxidizing enzyme- and thermally-reduced-graphene-oxide-(RGO)-based amperometric biosensor for direct evaluation of L-glutamate in biological media such as brain extracts or serum.

### BACKGROUND ART

Dopamine (DA) controls many vital physiological functions and is critically involved in several neuropsychiatric disorders. Pathological mechanisms of DA action related to the glutamate system. L-glutamate is known as the most common excitatory neurotransmitter in the mammalian central nervous system (CNS) [1]. Abnormal transmission of L-glutamate can cause neurological diseases such as communication dysfunction, cognitive impairments, schizophrenia, Parkinson's disease, stroke, and epilepsy [2-4]. Thus, investigations of L-glutamate release in living organisms could help to identification of novel, L-glutamate-related pathophysiological pathways. For these studies were used a newly developed strain of rats (DAT-knockout, DAT-KO rats) in which the gene encoding the DAT was disrupted by using zinc finger nuclease technology (ZFN) [5]. DAT-KO rats could provide a novel translational model for human diseases involving aberrant DA function and/or mutations affecting the DAT or related regulatory mechanisms. Thus, investigations of L-glutamate release in rats and in mice brain extracts could help unveil the role of L-glutamate in DA transporter pathologies. Since metabolisms of glutamine/glutamate are closely interconnected and involved in a number of metabolic pathways like the formation of other amino acids, antioxidant glutathione, ammonia, and urea, the assessments of L-glutamate concentration may indicate disorders or diseases related to such bioconversion reactions [6].

Glutamate content usually was assessed using the microdialysis-HPLC method [7] and only a few electrochemical biosensors were designed. However, over the twenty years, there has been a dramatic increase in the development of amperometric L-glutamate biosensors due to their, sensitivity, real-time analysis, cost-effectiveness, and portability compared to conventional laboratory techniques such as fluorescence [8], gas chromatography-mass spectrometry [9,10], high-performance liquid chromatography [11] or ELISA [12]. Amperometric L-glutamate biosensors are generally developed using L-glutamate oxidase (GluOx) and Pt or Pt-modified electrodes [12]. This enzyme had 100% specificity for L-glutamate [13]. Meanwhile, Pt is the "gold standard" electrode because of its excellent catalytic activity towards hydrogen peroxide, the electroactive by-product of most enzymatic biosensors including L-glutamate [14]. The biosensor based on a pair of GluOx and Pt has several advantages, such as simple fabrication, good reproducibility, faster response time, and high sensitivity. Nonetheless, there are some disadvantages including a short lifetime, high operating voltage (mainly, the used potential is from 0.5 to 0.7 V vs Ag/AgCI), and a narrow linear detection range [15]. For an analytical method, the linear part of the calibration curve of the biosensor should cover the concentration at which the sample is usually analyzed. The concentration of L-glutamate in biofluids, such as plasma, whole blood, cerebrospinal fluid (CSF), saliva, and urine are in the range of 5-100 µM, 150-300 µM, 0.5-2 µM, 0.232 ± 0.177 µM, and 34-191 mM, respectively [12,15,16]. Thus, a wide linear range is a very important characteristic of biosensors for accurate detection and quantification of L-glutamate in various biofluids.

Nowadays, a growing body of literature examines in vivo L-glutamate biosensors which help understand the physiology of neurotransmitters in brains [17]. However, in vivo sensors are extremely invasive and can lead to further complications such as infections [12]. Moreover, the operational stability of in vivo biosensors usually are poor. Wei et al. reported that the biosensor's sensitivity decreased up to 46.32 ± 5.08% after 9 hours of in vivo measurements [18]. While fabricating in vitro L-glutamate biosensors the wide linear range, high sensitivity, good stability, and low operating voltage can be achieved.

Over the years, several electrochemical L-glutamate biosensors have been designed and patented: CN113655104 - Preparation method of the glutamic acid biosensor; GB2259771 - Electrochemical throughflow enzyme biosensor; CN111007125 - Preparation method of the glutamic acid biosensor; KR1020070099463 - Biosensor for sensitively detecting monosodium glutamate by comprising nanowire with excellent electrical characteristics and a method for preparing the same; CN111855777 - Glutamate oxidase biosensor as well as preparation method and application thereof; US20220218246 - Direct electron transfer glutamate biosensor using platinum nanoparticle and carbon nanotubes. Many of the L-glutamate biosensors are based on Glutamate oxidase and special materials such as gold nanoparticle/sulfhydryl compound, pre-oxidises Pt, cobaltosic oxide/carbon mixed ink, nanowires, PtNP-coated MXene-Ti3C2Tx solution, platinum nanoparticle and carbon nanotubes composition, etc. All of the above-mentioned analytic tools had some disadvantages including narrow linear detection range, short lifetime, and high operating voltage.

### BRIEF DESCRIPTION OF THE INVENTION

**Biosensor.** The disclosed construction of the biosensor can be employed for sensing different biochemical metabolites, while using their corresponding selective oxidizing enzymes immobilized on the surface of the biochemical sensor.

The biosensor generates an anodic current of the electro-oxidation reaction of a metabolite under investigation and the sensor's enzyme, thereby allowing to evaluate the presence and concentration of the investigated metabolite.

The present biosensor comprises a working electrode and an adjustable semi-permeable membrane, the membrane further comprising the selected/selective oxidizing enzyme immobilized with a stabilizing agent, and in composition with a carbonaceous material (preferably, thermally reduced graphene oxide (RGO)) added for the reduction of the biosensor's operating potential required for the electro-oxidation. An examples of the stabilising agent are albumin, collagen, fibrin, keratin or gelatin - an inert protein from which, by help of aldehyde or other crosslinker, a network is arranged into which the enzyme is incorporated: this stabilizes the enzyme. Such an albumin-immobilization-matrix can be used for stabilizing many enzymes; it is important to select the correct ratio of enzyme to albumin, and other immobilization conditions, such as buffer pH. The reduction of the operating potential (voltage) reduces influence of interfering compounds present in complex biological samples, due to ability of the biosensor to operate at low working potential.

The fully assembled biosensor device comprises: the membrane with the immobilized enzyme into RGO paste, the membrane is coupled with the working electrode via a contact zone, while the membrane is streched-out, for example, on a rubber O-ring, and inserted into a measuring cell, while being mounted onto the biosensor's body.

The biosensor's permeable enzyme-immobilized membrane (1) may be replaced. The disclosed electrochemical biosensor is easy to prepare, and calibrate, and has adequate stability.
**L-glutamate biosensor.** The biosensor for sensing L-glutamate generates an anodic current of the electrooxidation reaction of ammonia (NH₃) released during the reaction catalyzed by L-glutamate oxidase, while L-glutamate is converting to alfa-ketoglutarate.

The biosensor comprises a working graphite electrode and adjustable semipermeable membrane containing L-glutamate oxidase (EC 1.4.3.11) immobilized into albumin and thermally-reduced-graphene-oxide (RGO).

The addition of RGO into the enzymatic membrane of the biosensor allows for the reduction of the operating potential of the biosensor required for the hydrogen peroxide electrooxidation up to -0.1 V. Namely, the influence of interfering compounds such as ascorbic acid or uric acid, presenting in complex biological samples may be reduced due to ability of the biosensor to operate at low working potential.

The L-glutamate biosensor serves as an analytical tool possessing a wide analytical range, which can solve the problem of L-glutamate balance, i.e. - when L-glutamate is too much or too low.

The performance of the L-glutamate biosensor is approved in terms of the detection of L-glutamate in serum, bovine, and mice brain extracts.

### BRIEF DESCRIPTION OF THE DRAWINGS

Features of the invention believed to be novel and inventive are set forth with particularity in the appended claims. The invention itself, however, may be best understood by reference to the following detailed description of the invention, which describes exemplary embodiments, given in non-restrictive examples, of the invention, taken in conjunction with the accompanying drawings, in which:
- **Figure 1**: depicts the calibration curve and the linear range of L-glutamate biosensor GluOxRGO according to the invention obtained at potential-0.1 V vs Ag/AgCI in sodium acetate buffer solution (0.01 M; pH 5.0).
- **Figure 2**: depicts the current-time responses of the GluOxRGO biosensor on addition of 0.3 and 0.15 mM L-glutamate (Glu), 0.05 mM L-glucose (Glc), 2.5 µM ascorbic acid (AA), 1 µM uric acid (UA), 0.5 µM serotonin (Ser), 0.5 µM adenosine (AD), 0.5 µM dopamine (DA) in sodium acetate buffer solution (0.01 M; pH 5.0). E= - 0.1 V vs Ag/AgCI.
- **Figure 3**: depicts the concentration of L-glutamate in blood serum samples obtained using the biosensor GluOxRGO and by colorimetric method.
- **Figure 4**: depicts A) - the scheme of fully assembled biosensor according to the invention: a membrane (1) with immobilized enzyme into RGO paste, a graphite working electrode as a contact zone with enzyme membrane (2), a rubber O-ring (3), a measuring cell (4), the biosensor's corpus (body) (5), and B) - graphical representation of operation mechanism of the amperometric L-glutamate sensor based on GluOx- and RGO- functional membrane.

Preferred embodiments of the invention are described herein below with reference to the drawings. Each figure contains the same numbering for the same or equivalent element.

### DETAILED DESCRIPTION OF THE INVENTION

It should be understood that numerous specific details are presented to provide a complete and comprehensible description of the invention embodiment. However, the person skilled in the art will understand that the embodiment examples do not limit the application of the invention which can be implemented without these specific instructions. This description should not be considered to be constraining the invention to given embodiment examples but only as one of the possible implementations of the invention.

**Physical parameters of the biosensor.** The biosensor for metabolites measurements comprises at least a working electrode (2), and a permeable membrane (1) covering the surface of the working electrode (2), wherein the membrane (1) comprises an immobilized enzyme with a carbonaceous material in contact with the surface of the working electrode (2) for bioelectrocatalytic oxidation of said metabolite.

The permeable enzyme-immobilized membrane (1) preferably is made of terylene, polycarbonate, or polyester layer (membrane). The permeability of the membrane is an essential feature, for the metabolite under investigation to arrive through the membrane into the electrode. The effective (nominal) thickness of the membrane is in range from 10 µm -12 µm, while the pore size (diameter) may be selected from 0.4 µm to 0.45 µm.

The membrane (1), before its preparation, may comprise a crosslinking agent for a selected sensoric enzyme immobilization, the crosslinking agent is selected from aldehydes, carbodiimides, imidoesters, and pyrocarbonates. This is one of ways to immobilize a selected enzyme onto the surface of the membrane (1).

The stabilising agent is an inert protein from which, by help of aldehyde or other crosslinker, a network is arranged into which the enzyme is incorporated and stabilized. The stabilising agent can be albumin, or other proteins with functional groups suitable for binding - collagen, fibrin, keratin or gelatin.

The carbonaceous material comprised in the enzyme membrane (1) is used as electrode material for to enhance an electron transfer between enzyme active site and the electrode surface during bioelectrochemical reaction. This carbonaceous material improves electrochemical reactions by serving as an electrode material with a large surface area due to the porosity and functional groups present. The carbonaceous material component is obtainable after reduction of graphene oxide. Reduced graphene oxide (RGO) can be synthesized in different methods (chemically or thermally), and the properties of the product (morphology and resulting functional groups) depend on the method used. One preferred example of the carbonaceous material is thermally reduced graphene oxide (RGO).

The working electrode (2) material may be selected from graphite, silver, and platinum.

The biosensor's permeable enzyme-immobilized membrane (1) is replaceable, or removable. The spare enzymatic membranes (1) can be stored in separate place, for example, refrigerator, and set up with the biosensor and its working electrode (2) when needed, for example, the measurement characteristics of the membrane (1) currently used in measurements became worse.

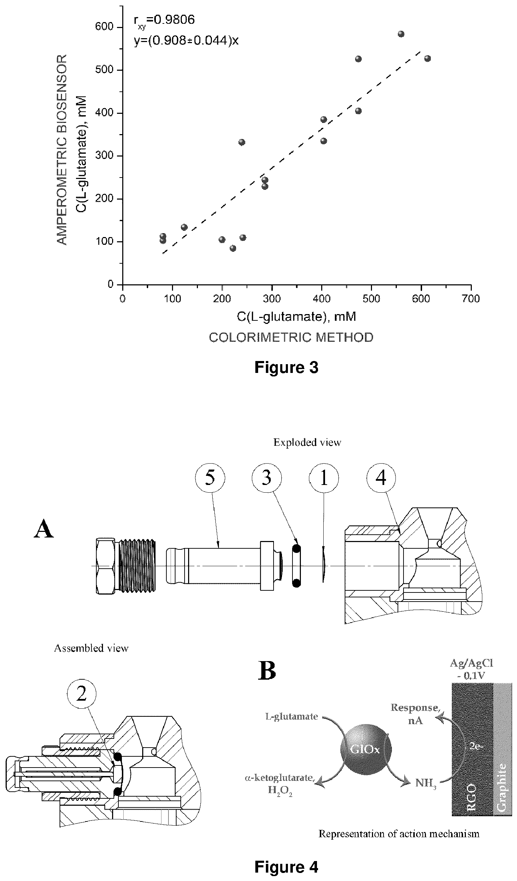

A physic implementation of the biosensor device is presented in Figure 4. It comprises: the membrane (1) with the immobilized enzyme into RGO paste, where the enzymatic membrane (1) is coupled with the working electrode (2) via a contact zone. The membrane (1) is streched-out, for example, using a rubber O-ring (3) and fixing the membrane (1) to this ring (3). Further, the membrane (1) with the ring (3) is mounted to the biosensor's body (5) providing the contact with the working electrode (2). The prepared biosensor (5) with the membrane (1) is inserted into a measuring cell (4) which then is filled with a biological sample under investigation.

**L-glutamate biosensor.** The amperometric biosensor for sensing L-glutamate and comprising the functional (sensing/oxidizing) layer of (GluOxRGO) is easy to prepare, and calibrate, and has adequate sensitivity and stability. The biosensor detects current generated during the electrooxidation of NH₃ released in the L-glutamate converting to the alfa-ketoglutarate reaction catalyzed by L-glutamate oxidase (Figure 4) at working electrode (2) potential of - 0.1 V vs. Ag/AgCl. The biosensor comprises a semipermeable membrane (1) with RGO and immobilized L-glutamate oxidase, a working graphite electrode (2) serving as a contact zone, and an isolating corpus (body) (5) of the biosensor (Figure 4).

**Preparation of the membrane for L-glutamate.** A simple and effective method to prepare an enzymatic membrane (1): first, a semi-permeable terylene film is fixed on a rubber ring (3) (inner diameter of 3 mm). 5 µl of mixture containing L-Glutamate Oxidase, bovine serum albumin in PBS, glutaraldehyde and RGO powder with the pasting liquid consisting of 10% polyvinyl dichloride in acetone was deposited on the inner surface of the ring-fixed membrane (1) and then left at 4 °C overnight. The mixture deposited onto the inner surface of the membrane (1), preferably, comprises the proportions of compontents, by weight: 1 part of GluOx : 1 part of BSA : 4 parts of PBS : 2 parts of RGO : 1 part of GA.

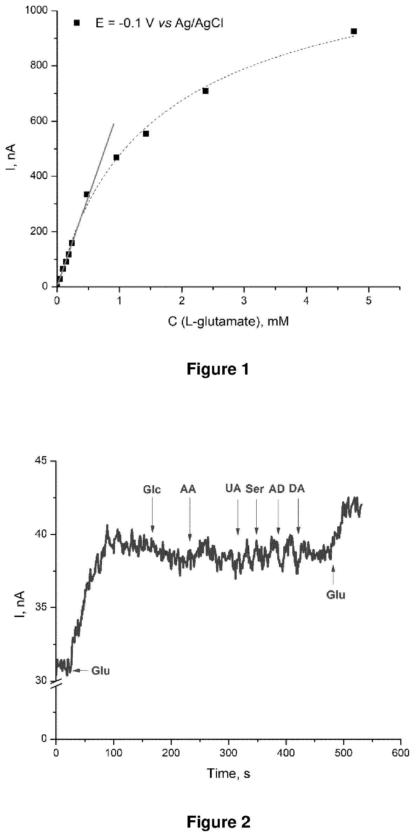

**Experiments.** A custom-made potentiostat (Vilnius University, Life Sciences Center, Institute of Biochemistry) with a conventional three-electrode system composed of a platinum plate auxiliary electrode, a saturated Ag/AgCl reference electrode, and a working electrode (biosensor) was used for chronoamperometric experiments. The chronoamperometric measurements were carried out in a thermostatic electrochemical cell (volume 1 ml) at 20 ⁰C. The current-time response of the biosensor to L-glutamate at working potentials - 0.1 V, vs. Ag/AgCI was recorded in stirred 10 mM sodium phosphate (PBS) or acetate buffer solutions containing 100 mM of KCI, and pH = 7.2 and pH = 5.0, respectively. The L-glutamate concentration in brain samples was determined from the linear part of the calibration curve presented in Figure 1. The calibration curve is obtained experimentally by adding 0.05, 0.1, 0.15, 0.2, 0.25, 0.5, 1.0, 1.5, 2.5, and 5 mM of L-glutamate into an electrochemical cell using an automatic pipette (Eppendorf research, Hamburg, Germany). The concentration of L-glutamate measurements in serum or brain samples were performed at the Bioanalysis department, Institute of Biochemistry, Life Science Center of Vilnius University by a newly created biosensor.

To verify the specificity of the GluOxRGO/Pt biosensors response towards L-glutamate, common interfering agents such as 0.05 mM L-glucose (Glc), 2.5µM ascorbic acid (AA), 1 µM uric acid (UA), 0.5 µM serotonin (Ser), 0.5 µM adenosine (AD) and 0.5 µM dopamine (DA) was used. The biosensor showed a rather negligible signal to the addition of these analytes, indicating an unaffected response to L-glutamate. The current responses to 0.5 and 0.25 mM of L-glutamate were recorded before and after the addition of other analytes and the biosensor did not show any notable change in current-time response (Figure 2). These results suggest that exposure to other neurochemicals would not affect the performance of the L-glutamate biosensor.

The analytical accuracy of the biosensor was confirmed by determining the L-glutamate concentrations by the biosensor and comparing their values with the values determined by an approved colorimetric L-glutamate measurement method. The method is based on an enzymatic assay, which results in a colorimetric (450 nm) product, proportional to the L-glutamate present in a sample. The measurement consists of two steps: sample preparation and colorimetric enzymatic reaction. Sample preparation was carried out using a modified procedure as described [20]. Briefly, the sample was sonicated for 30 minutes to achieve a homogeneous mixture. Afterwards, 100 µL of the sample was placed in 900 µL of standard measurement buffer solution (50 mM Na₂HSO₄ pH 7.4) and heat-inactivated at 85 °C for 30 min. Afterwards, the sample was cooled down to room temperature and centrifuged at 13 000 g for 15 min. Finally, the 50 µL of supernatant was removed and used for L-glutamate measurements. The measurement was conducted according to the standard procedure developed by Sigma [23] following the technical bulletin. A 96-well flat-bottom plate was used for measurement. A calibration curve was set up by placing 92 µL of L-glutamate assay buffer, 8 µL of L-glutamate developer solution, 2 µL L-glutamate enzyme mix, and a respective volume of L-glutamate standard in different wells. The total volume was 150 µL and was achieved by additionally placing a required volume of L-glutamate assay buffer in the wells. Samples were measured using a similar procedure, up by placing 92 µL of L-glutamate assay buffer, 8 µL of L-glutamate developer solution, 2 µL L-glutamate enzyme mix, and 50 µL of sample in different wells. The reaction mixture was incubated at 37 °C for 30 minutes and the absorbance at 450 nm was measured and compared to the control. The concentration of L-glutamate was received from the calibration curve. The calibration curve and all the samples were measured in duplicate.

The colorimetric analysis results of L-glutamate compared with results obtained using the biosensor are presented in Figure 3. Data indicates, that L-glutamate concentrations determined using the L-glutamate biosensor, are slightly lower than those of colorimetric analysis. These findings suggest that, in general, the response of the biosensor is not influenced by interfering substances and generates a correct response to L-glutamate in serum samples.

The accuracy of analysis in biological samples such as blood serum, bovine, mice, and rat brain extracts also have been assessed by adding known concentrations of L-glutamate into the samples and by following the detection of the real concentration of L-glutamate according to the registered response of the biosensor (Table 1).

**Table 1. Comparison of the added amount of L-glutamate and obtained concentrations using L-glutamate biosensor in serum, bovine, and rat brain samples.**

| **L-glutama te added, mM** | **Bovine brain** | | | **Mice Brain** | | | **Serum** | | |
|---|---|---|---|---|---|---|---|---|---|
| | L-glutamat e found, mM | Recove ry, % | RSD , % | L-glutamat e found, mM | Recove ry, % | RS D, % | L-glutamate found, mM | Recove ry, % | RS D, % |
| **0.048** | 0.049 | 102 | 5.4 | 0.052 | 108 | 3.8 | 0.047 | 97 | 6.2 |
| 0.099 | 0.109 | 110 | 6.9 | 0.095 | 96 | 5.3 | 0.095 | 96 | 4.2 |
| 0.143 | 0.166 | 116 | 12.7 | 0.159 | 111 | 6.5 | 0.171 | 119 | 5.3 |

The L-glutamate biosensor demonstrated rather good recovery. The recovery rate in the range of 102-116 %, 96-111 %, and 96-119 % RSD from 5.4 to 12.7 %, 3.8-6.5 %, and from 4.2 to 6.2 % was calculated in bovine, mice brains, and serum, respectively. These findings suggest that the biosensor generates a correct response to L-glutamate in biological samples since operates at a low electrode potential of -0.1 V.

Storage stability of the GluOxRGO biosensor showed that 90 % of the residual activity remained after 3 months of refrigerated storage. Operational stability was investigated by assessing responses to 2 mM of L-glutamate during 2 weeks. The activity of the biosensor decreased by about 20 % after 14 days.

Materials for creation of L-glutamate biosensor: L-Glutamate Oxidase from Streptomyces sp., Merck KGaA. Brain Extract from bovine brain, Type VII, Merck KGaA, Germany. Mice and rat brain extracts were obtained from the Department of Neurosciences, University of Mons, Belgium. Bovine serum albumin (BSA), Merck KGaA, Germany. Semi-permeable terylene membrane (thickness 12 µm, pore diameter 0.4 µm), Joint Institute of Nuclear Research, Russia. L-glutamate Assay Kit, Merck KGaA, Germany. Other chemical reagents were obtained from Sigma-Aldrich and were of analytical grade unless otherwise mentioned. As default buffer 10 mM sodium acetate (pH=5.0) and phosphate buffer (PBS) (pH=7.2) solutions containing 0.01 M of KCI, were used. A stock solution of L-glutamate 1 M in PBS. Reduced graphene oxide (RGO) was synthesized from the natural graphite according to the protocol reported by leva Sakinyte's doctoral dissertation (Vilnius University, Life Sciences Center, Institute of Biochemistry). The sample obtained was denoted as RGO.

### Examples of use of the L-glutamate biosensor according to the invention:

### Example 1

The L-glutamate biosensor according to the invention was applied for the detection of L-glutamate concentration in bovine brain extract. First, the sensor was calibrated with solutions of the L-glutamate of known concentration, the calibration curve is shown in Figure 1.

The concentration of L-glutamate was obtained with the biosensor by following steps:
- the electrochemical cell was filled with 1 ml of 10 mM sodium acetate buffer solution containing 100 mM of KCI, pH = 5.0;
- 50 µL of brain homogenate samples were added into the cell using an automatic pipette;
- the response of the electrode (biosensor) at a working potential of -0.1 V *vs.* Ag/AgCI was recorded using the Advanced Electrochemical System PARSTAT 2273 (Princeton Applied Research, Oak Ridge, USA);
- the concentration of L-glutamate in the brain samples was calculated from responses of the biosensor using a calibration curve (Figure 1) where sensitivity to 1 mM of L-glutamate was ca. 7 µA/cm².

### Example 2

The L-glutamate biosensor according to the invention was applied for the detection of L-glutamate concentration in blood serum. First, the sensor was calibrated with solutions of the L-glutamate of known concentration, the calibration curve is shown in Figure 1.

The concentration of L-glutamate was obtained with the biosensor by following steps:
- the electrochemical cell was filled with 1 ml of 10 mM sodium acetate buffer solution containing 100 mM of KCI, pH = 5.0;
- 50 µL of serum samples were added into the cell using an automatic pipette;
- the response of the electrode (biosensor) at a working potential of -0.1 V *vs.* Ag/AgCI was recorded using the Advanced Electrochemical System PARSTAT 2273 (Princeton Applied Research, Oak Ridge, USA);
- the concentration of L-glutamate in the serum samples was calculated from responses of the biosensor using a calibration curve (Figure 1) where sensitivity to 1 mM of L-glutamate was ca. 7 µA/cm².

A good correlation was found between the described method and the commercial colorimetric method; the correlation coefficient was 0.9806 (n = 20) (Figure 3).

### REFERENCES: NON-PATENT LITERATURE

**1.** McCutcheon, R.A.; Krystal, J.H.; Howes, O.D. Dopamine and glutamate in schizophrenia: biology, symptoms and treatment. World Psychiatry 2020, 19, 15-33.
**2.** Coyle, J.T. Glutamate and Schizophrenia: Beyond the Dopamine Hypothesis. Cell Mol Neurobiol 2006, 26, 363-382.
**3.** Fern, R.; Matute, C. Glutamate receptors and white matter stroke. Neurosci Lett 2019, 694, 86-92.
**4.** Lau, A.; Tymianski, M. Glutamate receptors, neurotoxicity and neurodegeneration. Pflug Arch Eur J Phy 2010, 460, 765-780.
**5.** Leo, D.; Sukhanov, I.; Zoratto, F.; Illiano, P.; Caffino, L.; Sanna, F.; Messa, G.; Emanuele, M.; Esposito, A.; Dorofeikova, M.; Budygin, E.A.; Mus, L.; Efimova, E.V.; Niello, M.; Espinoza, S.; Sotnikova, T.D.; Hoener, M.C.; Laviola, G.; Fumagalli, F.; Adriani, W.; Gainetdinov, R.R. Pronounced hyperactivity, cognitive dysfunctions, and bdnf dysregulation in dopamine transporter knockout rats. The Journal of Neuroscience 2018, 38, 1959-1972.
**6.** Sandstrom, P.; Trulsson, L.; Gasslander, T.; Sundqvist, T.; Von Dobeln, U.; Svanvik, J. Aerum amino acid profile in patients with acute pancreatitis. Amino Acids 2008, 35, 225-231 .
**7.** Zieminska, E.; Toczylowska, B.; Diamandakis, D.; Hilgier, W.; Filipkowski, R.K.; Polowy, R.; Orzel, J.; Gorka M.; Lazarewicz, W. Glutamate, glutamine and GABA levels in rat brain measured using MRS, HPLC and NMR methods in study of two models of autism. Front Mol Neurosci 2018, 11, 418.
**8.** 7 Chapmanustin, J.; Zhou, M. Microplate-based fluorometric methods for the enzymatic determination of I-glutamate: application in measuring I-glutamate in food samples. Anal chim acta 1999, 402, 47-52.
**9.** Nozal, M.J.; Bernal J.L.; Toribio, M.L.; Diego, J.C.; Ruiz, A. Rapid and sensitive method for determining free amino acids in honey by gas chromatography with flame ionization or mass spectrometric detection. J Chromatogr A 2004, 1047, 137-146.
**10.** Shina, H.J; Park, N.H.; Lee, W.; Choi, M.H.; Chung, B.C.; Hong, J. Metabolic profiling of tyrosine, tryptophan, and glutamate in human urine using gas chromatography-tandem mass spectrometry combined with single SPE cleanup. J Chromatogr B 2017, 1051, 97-107.
**11.** Zhang S.; Takeda, Y.; Hagioka, S.; Takata, K.; Aoe, H.; Nakatsuka, H.; Yokoyama, M.; Morita, K. Measurement of GABA and glutamate in vivo levels with high sensitivity and frequency. Brain Res Protoc 2005, 14, 61-66.
**12.** Schultz, J.; Uddin, Z.; Singh, G.; Howlader, M.M.R. Glutamate sensing in biofluids: recent advances and research challenges of electrochemical sensors. Analyst 2020, 145, 321-347*.*
**13.** Jamal, M.; Hasan, M.; Mathewson, A.; Razee, K.M. Disposable sensor based on enzyme-free Ni nanowire array electrode to detect glutamate. Biosens Bioelectron 2013, 40, 213-218.
**14.** Tan, C.; Yin, H.; Brooks, V.; Arumugam, P.U.; Siddiqui, S. A study of the effect of electrochemical roughening of platinum on the sensitivity and selectivity of glutamate biosensors. J Electrochem Soc 2022, 169, 037510.
**15.** Hawkins, R.A. The blood-brain barrier and glutamate. Am J Clin Nutr 2009, 90, 867S-874S.
**16.** Jasim, H.; Carlsson, A.; Hedenberg-Magnusson, B.; Ghafouri, B.; Ernberg, M. Saliva as a medium to detect and measure biomarkers related to pain. Sci Rep 2018, 8, 3220*.*
**17.** Hamdan, S.K.; A.M. Zain. In vivo electrochemical biosensor for brain glutamate detection: a mini-review. Malays J Med Sci 2014, 21, 12-26.
**18.** Wei, W.; Song, Y.; Wang, L.; Zhang, S.; Luo, J.; Xu, S.; Cai, X. An implantable microelectrode array for simultaneous L-glutamate and electrophysiological recordings in vivo. Microsyst Nanoeng 2015, 1, 15002.
**19.** Gineityte, J.; Meškys, R.; Dagys, M.; Ratautas, D. Highly efficient direct electron transfer bioanode containing glucose dehydrogenase operating in human blood. *J Power Sources* **2019,** *441,* 227163.

## Claims

1. A biosensor for metabolite measurements in biological samples, the sensor comprising at least
- a working electrode (2), and
- a layer with an immobilized enzyme responsible for selectivity of the biosensor to a selected metabolite,
**wherein** the biosensor comprises a permeable membrane (1) covering the surface of the working electrode (2), wherein the membrane (1) comprises a layer of the immobilized enzyme with a carbonaceous material in contact with the surface of the working electrode (2) for bioelectrocatalytic oxidation of said metabolite.

2. The biosensor according to claim 1, **wherein** the permeable enzyme-immobilized membrane (1) is a terylene, polycarbonate, or polyester membrane.

3. The biosensor according any of claims 1 to 2, **wherein** the permeable enzyme-immobilized membrane (1) has nominal thickness from 10 to 12 µm and pore size from 0.4 to 0.45 µm.

4. The biosensor according to any one of claims 1 to 3, **wherein** the enzyme membrane (1) for enzyme immobilization further comprises an enzyme-stabilizing agent and a crosslinking agent, wherein the crosslinking agent is selected from aldehydes, carbodiimides, imidoesters, and pyrocarbonates, and the enzyme-stabilizing agent is selected from albumin, collagen, fibrin, keratin or gelatin.

5. The biosensor according to any one of claims 1 to 4, **wherein** the carbonaceous material in the enzyme membrane (1) is thermally reduced graphene oxide (RGO).

6. The biosensor according to any one of claims 1 to 5, **wherein** the working electrode (2) material is selected from graphite, silver, and platinum.

7. The biosensor according to any one of claims 1 to 6, **wherein** the permeable enzyme-immobilized membrane (1) mounted on the biosensor is replaceable.

8. The biosensor according to any one of claims 1 to 7, **wherein** the sensor comprises:
- the membrane (1) with the immobilized enzyme into RGO paste, wherein the enzymatic membrane (1) is coupled with the working electrode (2) via a contact zone, wherein the membrane (1) is fixed to a rubber O-ring (3);
- the membrane (1) with the ring (3) is mounted to the biosensor's body (5) providing the contact with the working electrode (2), and
- the prepared biosensor (5) with the membrane (1) is inserted into a measuring cell (4) fillable with a biological sample under test.

9. The biosensor according to any one of claims 1 to 8, **wherein** the metabolite selective by the biosensor is L-glutamate, and the immobilized enzyme on the membrane (1) is an oxidoreductase which oxidases the L-glutamate.

10. The biosensor according to claim 9, **wherein** the oxidoreductase oxidising the L-glutamate is L-Glutamate Oxidase.

11. The biosensor according to claim 9, **wherein** the working electrode (2) material is made of silver.

12. A method of preparing the membrane (1) of the biosensor for L-glutamate according to any of claims 9 to 11, comprising steps of
• fixing the semi-permeable terylene film on a rubber ring (3), preferably, having the inner diameter of 3 mm;
• depositing a mixture comprising L-Glutamate Oxidase, bovine serum albumin (BSA) in Phosphate buffer solution (PBS), glutaraldehyde (GA) and RGO powder with the pasting liquid comprising 10% polyvinyl dichloride in acetone, on the inner surface of the ring-fixed membrane (1);
• leaving the mixture-deposited membrane (1) for overnight at 4 °C.

13. The method of according to claim 12, **wherein** the mixture deposited onto the inner surface of the membrane (1) comprises the proportions of compontents, by weight: 1 part of GluOx : 1 part of BSA : 4 parts of PBS : 2 parts of RGO : 1 part of GA.

14. A method of measuring L-glutamate with the biosensor according to any claims of 9 to 11, **wherein** the applied working electrode (2) potential to the biosensor is not greater than 0.1 Volts vs. Ag/AgCl.

15. The method according to claim 14, **wherein** before the measurements in biological samples, the L-glutamate biosensor is calibrated with L-glutamate solutions of the known L-glutamate concentration.
